# EUROPEAN PATENT APPLICATION

(11) **EP 4 628 213 A1**
(43) Date of publication of application: **08.10.2025**
(21) Application number: 24176263.2
(22) Date of filing: 16.05.2024
(51) Int. Cl.: B01L 3/00, G01N 33/543

(54) **DEVICE FOR TESTING ANALYTE IN LIQUID SAMPLE**

(30) Priority: 07.04.2024 CN 202410411686
(71) Applicant: Hangzhou Laihe Biotech Co., Ltd., Hangzhou Zhejiang 310051 (CN)
(72) Inventor: YE, Weiwei, Hangzhou, 310051 (CN)
(74) Representative: Piotrowicz, Pawel Jan Andrzej

(57) **Abstract**

The invention discloses a device for testing an analyte in a liquid sample, and the device includes: a sample chamber for receiving the liquid sample, where the sample chamber includes an opening and side walls enclosing a chamber; a detection chamber, where the detection chamber is in fluid communication with the sample chamber, the detection chamber is provided with the opening through which a testing element is inserted into the detection chamber, and the testing element has a testing area; and a first cover, where the first cover is used for sealing the opening of the detection chamber. The device can be used for testing the analyte in the liquid sample and especially suitable for home self-testing.

## Description

### CROSS-REFERENCE TO RELATED APPLICATIONS

This application claims priority to Chinese Patent Application, Application No.: 2024104116867, filed on April 7, 2024, and all disclosures of this application, including but not limited to the specification, claims, abstract and accompanying drawings of this application are incorporated by reference in their entirety as a part of this application.

### BACKGROUND OF THE INVENTION

### Field of the Invention

The invention belongs to the field of in vitro diagnosis and is used for testing an analyte in a liquid sample, and in particular, to a device for testing some analytes in a urine sample and the properties of urine.

### Description of the Related Art

In the detection field, a testing device is a common medical testing instrument, which is mostly used to detect test samples in stored liquids, such as urine, blood, sewage, semi-solid substances (which refers to liquid samples converted by any suitable method), and the like. A common testing tool for testing a liquid sample is a reagent strip. Usually, there are two ways for the reagent strip to obtain the liquid sample. One way is to put the reagent strip directly into the liquid sample, and in this case, the bottom of the reagent strip is in contact with the liquid sample, such that the reagent strip can obtain the liquid sample; the other way is a dropping method, that is, the liquid sample is absorbed by a dropper and other tools and then dropped to the reagent strip, such that the reagent strip can obtain the liquid sample. In these two common ways, the reagent strip is in local contact with the liquid sample; usually, it can only be tested for one item. When the test item is changed, it is necessary to change a different reagent strip.

In addition, when there are multiple test items on a test strip, especially when biochemical tests are used, it is desired that each testing area on the test strip will contact with the liquid sample, such that the liquid sample can have a same reaction time in each testing area and the testing results within the same time can be judged. The manual operation of the test is unsafe and unsanitary if used.

It is necessary to further improve an existing conventional device, so a convenient, hygienic and safe device is used for testing, and especially a chemical method is used for testing the analyte in the urine.

### BRIEF SUMMARY OF THE INVENTION

In order to overcome the disadvantages of the prior art, a testing device is provided and used for testing the content or presence or absence of an analyte in a liquid sample; and when testing needs to be performed, a testing element is allowed to be inserted into a detection chamber for testing and assaying.

In some embodiments, the testing element of the invention includes a testing area and a non-absorbent support carrier, and the testing area is provided on the carrier. In some embodiments, the testing area includes an absorbent carrier on which reagents such as chemical substance and immune reagent are pretreated. In some embodiments, the testing area can be moistened only by direct contact with the liquid sample, but not by the carrier to absorb a liquid. The carrier does not have water absorption capacity, so even if the carrier contacts with the liquid, the testing area can remain dry without contacting with the liquid. In some embodiments, when testing needs to be performed, the testing element is provided, placed or inserted into the detection chamber; in this case, the liquid sample in the detection chamber does not contact with the liquid sample, or the liquid cannot be indirectly absorbed by the carrier; or the testing area is not allowed to contact with the liquid sample through the carrier to absorb the liquid sample. In some embodiments, one or more testing areas are provided on the testing element, each testing area includes an absorbent material, and these testing areas are spaced apart on the non-absorbent carrier. In some embodiments, an analyte is tested in each testing area; if there are a plurality of testing areas, for example, 2, 3, 5, 10 and 12 testing areas, 2-12 different analytes can be tested at one time. In some embodiments, a test strip includes an area with the testing area and an area without the testing area, and the two areas are located on the non-absorbent carrier of the test strip. The area with the testing area is close to one end of the test strip while the area without the testing area is close to the other end of the test strip. In some embodiments, when the test strip is inserted into the detection chamber and before testing is performed, the area with the testing area on the test strip is close to an opening of the detection chamber while the area without the testing area is close to the bottom of the detection chamber and contacts with the liquid at the bottom of the detection chamber; in this case, the testing area does not contact with the liquid at the bottom of the detection chamber, and the testing area is dry in a first state. After the liquid is allowed to contact with the testing area, the testing area is wet in a second state; after such contact is kept for a specified time such as 1-60 seconds and 1-10 seconds, the testing area is in the second state; after the testing area contacts with the liquid, it is still necessary to allow the liquid to depart from the testing area, and the testing area is in a third state and wet, but does not contact with the liquid. In this case, substances in the testing area, for examples, chemical substances, react with the analyte in the liquid sample. In a reaction process, the testing area presents color, and testing results can be judged by comparing the concentration or amount of the colors in the testing areas with a standard colorimetric card.

In some embodiments, the device includes a sample chamber for receiving the liquid sample, where the sample chamber includes an opening and transparent side walls enclosing a chamber; a detection chamber, where the testing element is used for inserting the detection chamber, and the detection chamber is in fluid communication with the sample chamber; when the liquid sample is accommodated in the sample chamber, a part of the liquid sample flows into the detection chamber. In some embodiments, after the liquid flows into the detection chamber, the testing element is inserted into the detection chamber. Of course, in some optional embodiments, the testing element is provided in the detection chamber, and then the sample chamber is allowed to accommodate the liquid sample. Therefore, at the beginning, the detection chamber does not contain the detection chamber, and when the liquid is accommodated in the detection chamber, the test strip is inserted into the detection chamber.

In some embodiments, the detection chamber is in fluid communication with the sample chamber through a channel. In some embodiments, the detection chamber is located on the outer wall of the sample chamber, and a channel is provided in a common wall of the detection chamber and sample chamber. When the liquid sample flows into the sample chamber, a part of the liquid sample also flows into the detection chamber. In some embodiments, the channel is provided in the bottoms of the detection chamber and sample chamber, and when the liquid sample is collected in the sample chamber, a part of the liquid sample flows into the detection chamber.

In some embodiments, a first cover and a second cover are provided, where the first cover is used for sealing the opening of the detection chamber, and the second cover is used for sealing the opening of the sample chamber. Therefore, the detection chamber includes an opening for inserting the testing element into the detection chamber, and the sample chamber includes an opening for receiving the liquid sample. When the liquid sample is collected in the sample chamber, the second cover is used for sealing the opening of the sample chamber. In some embodiments, after the testing element is inserted into the detection chamber, the opening of the detection chamber is sealed by the first cover. In some embodiments, liquid samples and air are both accommodated in the sample chamber and the detection chamber. The test strip is inserted into the detection chamber in an initial state, the testing area is not in contact with the liquid, but in the space containing air. When the liquid needs to contact with the liquid sample, the testing device is inverted, inclined or placed in other manners, such that the liquid in the detection chamber is located in the opening to contact with the testing area. In some embodiments, the detection chamber has the bottom and the opening; when the test strip is inserted into the detection chamber, the testing area is close to the opening, and the liquid sample is located in the bottom space of the detection chamber. In some embodiments, the liquid sample does not contact with the testing area. In some embodiments, after the liquid is expected to contact with the testing area and the liquid sample is expected to depart from the testing area, the device is allowed to return to the initial state again, for example, in an upright state. In this case, the liquid in the detection chamber returns to the bottom of the detection chamber.

In some embodiments, the carrier is provided in the detection chamber and the testing element is allowed to lean against the carrier. In some embodiments, a groove is provided in the carrier; and when the testing element is inserted into the detection chamber, the testing element is located in the groove. In some embodiments, the carrier has a diversion area near the opening of the detection chamber to guide the insertion of the testing element into the groove of the carrier. In some embodiments, the diversion area includes an inlet communicating with the groove, and when the testing element is inserted through the inlet, the testing element is also located in the groove. In some embodiments, the size of the inlet of the diversion area matches the thickness of the test strip. In some embodiments, the diversion area includes an inclined plane, and a bottom of the inclined plane is the inlet for inserting the test strip into the groove. In some embodiments, the groove faces a side wall of the detection chamber, and the groove and the side wall of the detection chamber form a narrow slit. In some embodiments, the side wall is transparent, and the testing results of the testing element can be read through the transparent side wall. In some embodiments, the groove has a depth, forms a narrow slit with the side wall of the detection chamber, and the test strip is located in the groove. When the liquid moves from the bottom of the detection chamber to the opening of the detection chamber, the test strip is still kept in the groove without any movement. In some embodiments, the carrier has two ends, one end thereof is close to the opening of the detection chamber, the other end thereof is close to the bottom of the detection chamber, and there is a distance from the other end to the bottom of the detection chamber, and the distance is similar to one opening or one hole, such that the liquid can flow into the narrow slit through the hole.

Further, the invention provides a method for testing an analyte in a liquid sample, and the method includes: providing a device, where the device includes a sample chamber for receiving the liquid sample, and the sample chamber includes an opening and side walls enclosing a chamber; a detection chamber, where the detection chamber is in fluid communication with the sample chamber, the detection chamber is provided with the opening through which a testing element is inserted into the detection chamber, and the testing element has a testing area; and a first cover, where the first cover is used for sealing the opening of the detection chamber; allowing the sample chamber to collect the liquid sample such that a part of the liquid sample flows into the detection chamber; and inserting the testing element through the opening of the detection chamber, where after the testing element is inserted, the liquid sample is not allowed to contact with the testing area and the testing area is allowed to be kept dry.

In some embodiments, after the test strip is inserted into the detection chamber, the liquid sample is allowed to directly contact with the testing area of the testing element, and after a period of contact, the liquid is allowed to depart from the testing area.

In some embodiments, the testing area on the test strip has a first state, a second state in which the testing area is moistened by contact with the liquid, and a third state after the liquid departs from the testing area.

In some embodiments, after the test strip is inserted into the detection chamber through the opening of the detection chamber, the opening of the detection chamber is sealed by a first cover. When the liquid sample is diverted into the detection chamber through the opening of the sample chamber, the opening of the sample chamber is sealed with a second cover. In some embodiments, when it is desired to allow the liquid sample to contact with the testing area, the device is inverted or inclined such that the liquid in the testing area moves and contacts with the testing area. After the liquid contacts with the testing area, the testing device is returned to an upright posture from an inverted or inclined position.

In some embodiments, one channel is provided in the bottoms of the detection chamber and sample chamber; and when the liquid sample is collected in the sample chamber, a cover of the detection chamber seals the opening of the detection chamber. In this case, the volume of the liquid flowing into the detection chamber is constant. Of course, when the sample chamber is used for collecting the liquid sample, the opening of the detection chamber is not sealed, and the liquid in the detection chamber has an equal level to the liquid in the sample chamber. In some embodiments, after the testing element is inserted into the detection chamber through the opening of the detection chamber, the level of the liquid in the detection chamber is below the testing area without contacting with the testing area. Then, the liquid in the detection chamber is moved from the bottom to the opening through inversion, such that the testing area is immersed in the liquid sample. In some embodiments, the time that the testing area is immersed in the liquid sample can be any time, such as 1 seconds - 1 minute, 1 minute - 5 minutes, generally 1-10 seconds or 1-30 seconds. In some embodiments, after the liquid is allowed to contact with the testing area, it is necessary to allow the liquid to depart from the testing area, instead of allowing the testing area to be immersed in the liquid sample for a long time. Herein, the main reason is that when chemical substances treated in the testing area contact with the analyte, the color of the chemical substances changes, or some colored substances are generated and are easily dissolved or diluted by the liquid sample, resulting in false negative results.

In some embodiments, the liquid sample is urine, and the analyte is one or more of the analytes in the urine, for example, hemameba or leukocyte, erythrocyte, urobilinogen, urine vitamin c, urine crystal, urine specific gravity, urine albumin, urine ketone bodies, urine colony count, urine pH, and nitrous acid. In some embodiments, positions where color change occurs are bottoms, and these bottoms can change in color and are all pretreated on the testing area. Because the testing area contains the absorbent materials, these reagents are treated on the testing area and used in the testing area after dried.

### BRIEF DESCRIPTION OF THE DRAWINGS

FIG. 1 is a schematic diagram showing a three-dimensional structure of a detection structure (including a test strip) according to a specific embodiment of the invention.
FIG. 2 is a schematic diagram showing an exploded structure of a device according to the invention.
FIG. 3 is a schematic diagram showing a three-dimensional structure of a combined product according to a specific embodiment of the invention (a testing element is inserted into a detection chamber).
FIG. 4 is a cross-section structure entity of a device according to a specific embodiment of the invention.
FIG. 5 is a schematic diagram showing a three-dimensional structure of a sample chamber and a detection chamber according to a specific embodiment of the invention, where the detection chamber does not include a testing element.
FIG. 6 is a schematic diagram showing a cross-section structure of a sample chamber and a detection chamber according to a specific embodiment of the invention, where the detection chamber does not include a testing element.
FIG. 7 is a schematic diagram showing a three-dimensional structure of a carrier for bearing a test strip (including a testing element) according to a specific embodiment of the invention.
FIG. 8 is a schematic diagram showing a three-dimensional structure of a carrier for bearing a test strip (excluding a testing element) according to a specific embodiment of the invention.
FIG. 9 is a schematic diagram showing a three-dimensional structure of a carrier for bearing a test strip according to a specific embodiment of the invention.
FIG. 10 is a schematic diagram showing a cross-section structure in a first step of a testing step according to a specific embodiment of the invention.
FIG. 11 is a schematic diagram showing a cross-section structure in a second step of a testing step according to a specific embodiment of the invention.
FIG. 12 is a schematic diagram showing a cross-section structure in a third step of a testing step according to a specific embodiment of the invention.
FIG. 13 is a schematic diagram showing a cross-section structure (inclined) in a fourth step of a testing step according to a specific embodiment of the invention.
FIG. 14 is a schematic diagram of inclination of a detection chamber according to a specific embodiment of the invention.

### DETAILED DESCRIPTION OF THE INVENTION

### Detection

Detection means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein or a polymer. In addition, detection means that the amount of a substance or material is tested. Further, assay also means immunoassay, chemical assay, enzyme assay, and the like.

### Samples

Samples that can be tested by the testing device of the invention include biological liquids (for example, case liquids or clinical samples). Liquid samples or liquid specimens may be derived from solid or semi-solid samples, including feces, biological tissues and food samples. The solid or semi-solid specimens may be converted to liquid specimens by any appropriate methods, such as mixing, mashing, macerating, incubating, dissolving, or digesting the solid specimens by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological sample is urine; and preferably, the biological sample is saliva, sputum, nasal secretion, or the like. Food samples include food processing substances, final products, meat, cheese, wine, milk, and drinking water. Plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other waste water.

In some embodiments, the sample of the invention may be a urine sample of human or mammal. Therefore, the device of the invention can be used for testing whether a liquid medicine is infected, or specific analytes in urine or the content of the analytes.

### Downstream and upstream

Downstream or upstream is divided according to a flow direction of a liquid. Generally, a liquid flows from an upstream area to a downstream area. The downstream area receives the liquid from the upstream area, and a liquid also may flow to the downstream area along the upstream area. Here, downstream or upstream is generally divided according to a flow direction of a liquid, for example, on some materials where capillary force is utilized to promote the flow of a liquid, a liquid may overcome gravity to flow towards an opposite direction to the gravity; and in this case, downstream or upstream is divided according to a flow direction of the liquid. For example, in the collection device of the present invention, in some preferred embodiments, the first chamber serves as a chamber for collecting a liquid sample, while the second chamber is in fluid communication with the first chamber, and the liquid flowing into the first chamber flows into the second chamber. The first chamber can be called upstream while the second chamber can be called downstream. Of course, such flow is natural flow of liquid under the action of gravity. Optionally, such natural flow is the flow of the liquid from the first chamber to the second chamber.

### Gas communication or liquid communication

Gas communication or liquid communication means that liquid or gas can flow from one place to another. In the flow process, the liquid or gas may pass through some physical structures that play a guiding role. The "pass through some physical structures" here means that the liquid passes through the surface of these physical structures or the internal space of these physical structures and flows to another place passively or actively, where the passive flow is usually caused by external forces, such as the flow under the capillary action. The flow here may also be a flow due to self-action (gravity or pressure) of the liquid or gas, and also may be a passive flow.

### Detachable combination

A detachable combination means that two components are connected in several different states or positional relationships. For example, with two components being physical components, they can be separated at the beginning and then connected or combined in an appropriate first case, and separated in an appropriate second case. Physically, such separation is spatial separation without contact. Alternatively, the two components are combined at the beginning, and can be physically separated from each other when appropriate. In short, combination or separation of two components or two objects can be easily made and repeated many times. Of course, the combination or separation can also be single-use. In addition, such combination can be a detachable combination between two components, or a two-by-two detachable combination between three or more components. For example, a first component, a second component, and a third component are provided, where a detachable combination is made between the first component and the second component or between the second component and the third component.

### Testing element

The testing element can be a lateral flow test strip that can detect a variety of analytes. Of course, other appropriate testing elements also can be used in the invention, and an element that can be used to detect whether a sample or a specimen contains an interested analyte may be called as the testing element. Such testing can be based on any technical principles, such as immunology, chemistry, electricity, optics, and physics. Various testing elements can be combined for use in the invention. One form of the testing elements is a test strip.

Test strips used in the invention may be commonly referred as lateral flow test strips. The specific structure and testing principle of the test strips are well known to a person skilled in the art in the prior art. A common test strip includes a sample collection area or a sample application area, a label area, a testing area and a water absorption area. The sample collection area includes a sample receiving pad, the label area includes a label pad, and the water absorption area may include a water absorbent pad. The testing area includes necessary chemical substances for detecting the presence or absence of the analyte, such as immunoreagents or enzyme chemical reagents. The nitrocellulose membrane test strip is commonly used, that is, the testing area includes a nitrocellulose membrane, and a specific binding molecule is immobilized on the nitrocellulose membrane to display the testing result; and other test strips such as cellulose acetate membrane or nylon membrane test strips may also be used. Of course, in the downstream of the testing area, there may also be a testing result control area. Generally, the control area and the testing area in the form of horizontal lines, namely, a test line or a control line. Such test strips are conventional. Of course, they may also be other types of test strips for detection under the capillary action. In addition, there are dry chemical reagent components on common test strips, for example, an immobilized antibody or other reagents. When the test strip contacts a liquid, the liquid flows along the test strip under the capillary action, and the dry reagent components are dissolved in the liquid and treated in a next area, and the dry reagents react in the area for necessary detection. The liquid flow mainly relies on the capillary action. An appropriate testing element according to the invention can be used to test any analyte. Preferably, the testing device of the invention is used to detect small drug molecules in saliva and urine. Of course, any samples of the above forms may be collected by the sample collector of the invention, regardless of being solid or liquid at the beginning, provided that these liquids or liquid samples flow to the collection chamber and the test strip is inserted from the cover of the sample collector for testing.

The test strips listed above rely on capillary forces to allow the liquid to flow from one end to the other end and pass through the testing area to complete the test of the analyte. There is also a test strip that allows the liquid to directly contact with the testing area without the capillary forces, so as to test the analyte. The so-called urine biochemical test strip is taken as an example. Therefore, in some embodiments, the test strip of the invention is made of a non-absorbent material as a supporting structure; there is an absorbent testing area on the non-absorbent material, and the absorbent testing area consists of the absorbent material; chemical substances are pretreated on the testing area and can react with the analyte to cause color change, thereby judging the presence or absence of the analyte or the form of the sample. Such reaction can be that the analyte directly reacts with a colored substrate to generate a new colored substance or the analyte reacts with some chemical substances to generate a new substance, and the new substance reacts with the colored substrate to generate a new colored substance. These substances may be selected from original colored substances generated in chemical reaction. These colored substances are precipitated and attached to the absorbent material on the testing area, such that the absorbent material can exhibit a specific color, and then the testing results can be judged through comparison of the specific color with a standard color card. Therefore, because the absorbent material is used as the testing area and provided on a non-absorbent support sheet, the liquid cannot flow from one end to the other, but the support sheet exerts its function to fix the testing area. If there are a plurality of testing areas, the plurality of testing areas are spaced apart and fixed onto the support sheet; when testing needs to be performed, it is necessary to allow the testing areas to directly contact with the liquid sample for testing and assaying.

Urine is taken as an example, and the PH value of the urine and the analyte in the urine can be tested, for example, hemameba or leukocyte, erythrocyte, urobilinogen, urine vitamin c, urine crystal, urine specific gravity, urine albumin, urine ketone bodies, urine colony count, urine pH, and nitrous acid. In some embodiments, any two or more of the above detection indexes can be selected for detection, so one or more absorbent materials, such as filter paper blocks, filter paper sheets and absorbent sheets, are arranged on the non-absorbent material as the supporting structure. Substantially, each absorbent material is considered as one testing area that includes an absorbent pad or an absorbent sheet, and an analyte is tested on each testing area. When it is desired that two or more kinds of analytes are tested on the testing area, two or more absorbent sheets are pasted on the non-absorbent material as the supporting structure, and the chemical substances are treated on the absorbent sheets and can react with two or more kinds of analytes in the sample and produce colors. These colored substances are precipitated on the corresponding absorbent blocks or absorbent sheets, and then the colors are compared by the standard color card to judge whether the corresponding analyte is positive or negative.

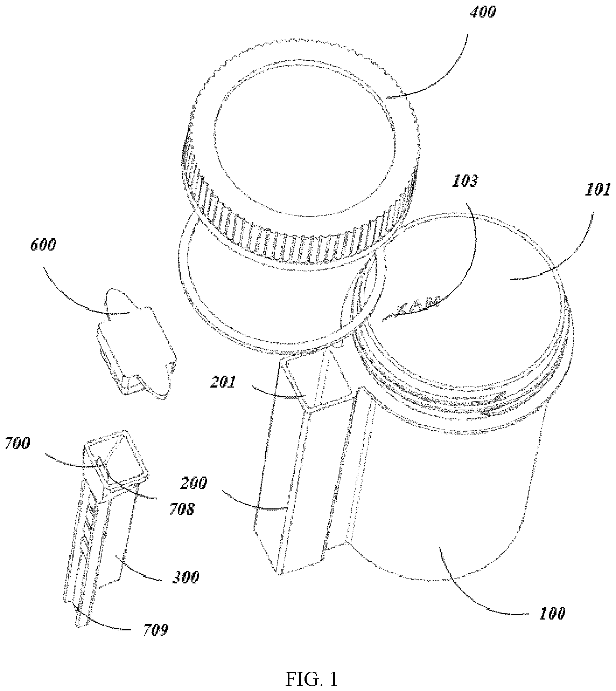

In some embodiments, the testing element or the test strip as shown in FIG. 1, FIG. 2, and FIG. 7 is taken as an example, there are four testing areas 702, 703, 704, 705 on one test strip 700; the analytes tested in these testing areas are different, and the four testing areas are all arranged on the non-absorbent sheet 701. Of course, the non-absorbent sheet can include one testing area, or it can include five, six or seven testing areas. These testing areas are arranged in sequence along the longitudinal axis of the test strip, and the analyte corresponding to each testing area can be printed or labeled on the support sheet 701. Of course, the analyte and the color blocks corresponding to the negative or positive of the analyte can also be labeled on the provided standard colorimetric card in a corresponding sequence on the test strip. Generally, the non-absorbent support sheet can be hard paper, and a plastic or metal sheet, while the absorbent testing area consists of the filter paper; the chemical substances are pretreated on the filter paper and dried to make filter paper blocks for testing; or these filter paper blocks are dried by a long strip of filter paper treated with the chemical substances and then pasted on the non-absorbent support sheet 55. These testing areas are arranged at specified intervals 706, 707, 708. These intervals are to prevent exchange of liquid between the testing areas and avoid inaccurate testing results caused by cross reaction. The "non-absorbent" material herein substantially means that when this material contacts with the liquid or water, it generally cannot absorb water by the capillary force to allow the liquid to flow on the material. For example, when one end 707 of the test strip is inserted into the water, the water cannot flow upward along the support sheet to pass through the testing area 706. Generally, the chemical substances are treated on the testing areas, and there are substrates that produce color in the reaction. These substrates are colorless or other colors at first, but their colors change through chemical reaction, for example, the substrates are colorless at first, but they will become other colors such as purple and red when reacting with the analyte. After the testing areas contact with the sample, it is desired that the sample is allowed to depart from the testing area to avoid the long-time immersion of the testing area in the liquid, and the colored substrate generated is dissolved or dispersed in the sample, resulting in false negative results.

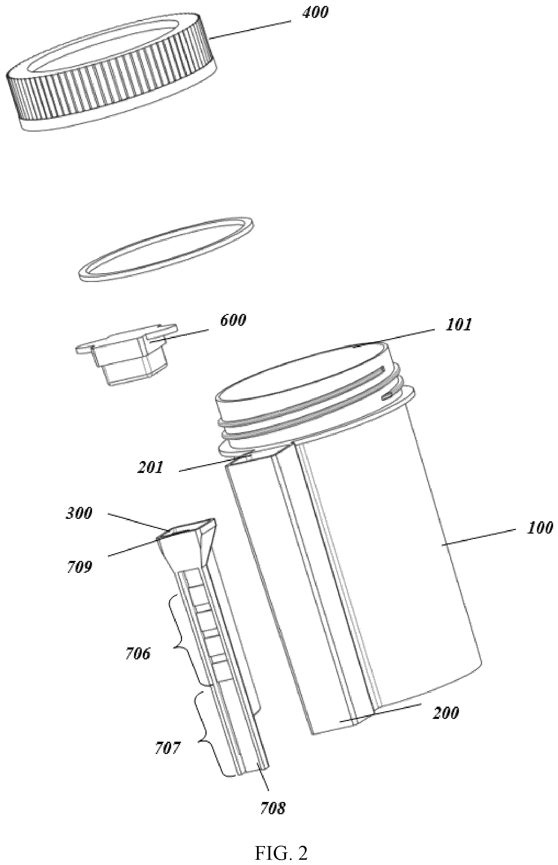

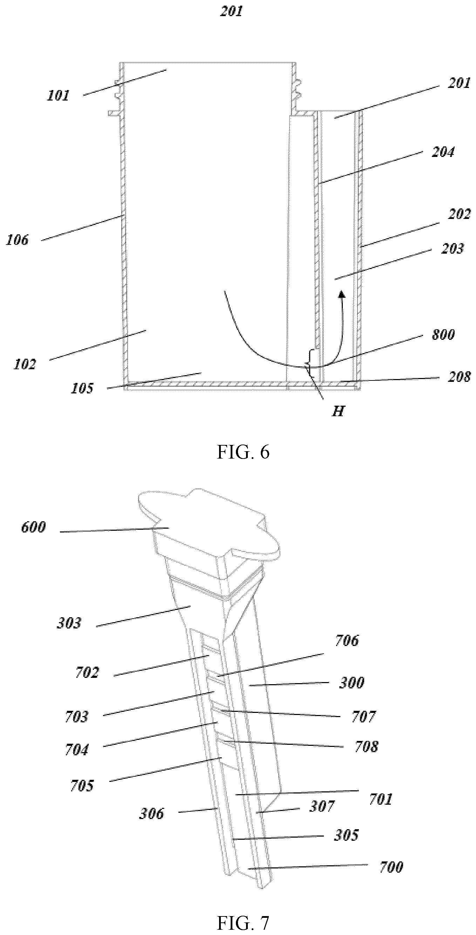

In some embodiments, as shown in FIG. 2 and FIG. 7, the test strip includes four testing areas 702, 703, 704, 705, where the analytes tested in the testing area 702 and the testing area 705 are the same; and similarly, the analytes tested in the testing area 703 and the testing area 704 are the same. The testing areas are spaced apart on the test strip 701, where the testing area 702 and the testing area 703 are arranged in sequence and the testing area 704 and the testing area 705 are arranged in sequence. One of such advantages is that the test strip needs to be inserted into the liquid sample; and when there is the few liquid sample in the collection chamber, the testing areas 704, 705 below the test strip can be moistened to complete testing. In addition, when there is the sufficient amount of the urine sample to moisten the four testing areas, it is enough to compare the testing results of one testing area with the colourimetric card. In addition, if an operator is not careful, no matter which direction the test strip is inserted into the carrier and one end of the test strip is immersed in the liquid sample, two testing areas are always kept dry before the test, such that testing can be successfully completed. The test strip also has the function, for example, in some cases, one of the four testing areas may be moistened in advance due to careless operation. For example, if the testing area 702 is moistened in advance by water, the testing area 704 can still be used for testing the analyte in urine, thereby ensuring that the test strip can complete the whole test without being wasted. For example, when one test strip 701 is pulled out from a box where the test strips are stored, one end of the test strip is generally held by hand and sometimes may fall off from the hand to an experimental table carelessly; there may be the liquid such as water on the experimental table, so individual testing areas on the test strip 701 will be moistened in advance, for example, the testing area 703 will be moistened in advance by water, and if the testing area 703 moistened in advance is used to contact with the urine, the testing area 703 moistened in advance contains water, and the saturated water absorption capacity of one testing area is customized. The so-called saturated water absorption capacity is the maximum volume of water absorbed by the testing area started from a dry state; once the testing area reaches the maximum volume, it can no longer absorb the water. When the absorbent material in the testing area absorbs enough water to reach the saturated water absorption capacity or absorbs water (without reaching the saturated water absorption capacity), if the absorbent material contacts with real liquid samples such as urine samples, the testing area cannot absorb more urine or absorbs some urine, meaning that the water for in advance moistening the testing area has a dilution effect on the urine; thus, the concentration of the analyte in the real urine in the testing area 703 is reduced, causing false negative results and even failing to react with the analyte in the urine. Generally, the urine needs to immediately depart from the testing area after instantaneously contacting with the testing area, but the testing area cannot be immersed in the liquid sample for a long time. The so-called instantaneous or short-time contact is about 1-2 seconds, 1-10 seconds, 10-30 seconds, and 30-60 seconds, and then the urine is allowed to depart from the testing area, such that the chemical substances on the testing area react with potential analytes in the urine staying in the testing area to generate colored substances and stay in the testing area. In this case, if a backup testing area 704 remains dry, the dry testing area 704 can still be allowed to contact with the urine, so as to obtain real testing results. In particular, when one test strip includes 5-10 or even more testing areas, one testing area should not be abandoned because it is moistened in advance; therefore, the test strip can be provided with two or more same testing areas as backup testing areas. The following will make detailed descriptions with reference to operations. Such arrangement can not only satisfy the operation of professionals, but also satisfy those home self-testers who have no operating experience to operate by themselves.

Such arrangement also has another advantage. For example, as shown in FIG. 7, the testing areas 702, 703, 704, 705 where same analytes are tested are arranged in sequence. For example, the analytes tested in the testing areas 702, 703 (such as hemameba (testing area 702) and nitrous acid (testing area 703)) are the same as the analytes tested in the testing areas 704, 705 (hemameba (testing area 704) and nitrous acid (testing area 705)). During testing, it is actually desired that the sample will depart from immediately after contacting the testing area, and the testing area cannot be immersed in the liquid sample all the time. Actually, two same testing areas of the testing areas are allowed to be located respectively at both ends of the test strip, or two testing areas and other two testing areas are respectively located at both ends of the test strip. When the device of the invention is used for testing, the test strip is allowed to be inserted into the detection chamber and kept in the detection chamber all the time; however, the detection chamber 200 is used for receiving the liquid sample (for example, for receiving the liquid sample from the sample chamber 100), if the liquid sample in the detection chamber is excessive and exceeds a maximum line 103 (MAX), for example, the level of the liquid exceeds the position of the testing area 705 on the test strip 701, the testing area 705 will always be immersed in the liquid during the test. With the testing area 705 being immersed in the liquid, the colored substance will be dissolved or dispersed in the urine in case of the colored substances, such that the colored substances will not be accurately precipitated on the testing area 705, and the colored substances on the testing area will be reduced, resulting in inaccurate testing results arising from false negative obtained through comparison of the colors of the colored substances with the external colorimetric card. However, in the invention, there is also a same testing area 703 located on the test strip 700, and the testing area 703 is located above the level of the sample; during the test, the liquid and the testing area 703 are allowed to immediately depart from urine after contacting with the urine, or the urine is allowed to immediately depart from the testing area 703 after contacting with the testing area, such that the analyte on the testing area 703 can be tested (same analytes are tested in the testing areas 703, 705). Therefore, for the test strip as shown in FIG. 7, as long as the urine is below the testing area 705, testing can be completed no matter what the test strip is inserted into the carrier and the chamber in any direction. If the test strip is inserted into the chamber in a direction opposite to a direction as shown in FIG. 7, no matter what any one or two (the lowest two testing areas near the liquid) of the four testing areas are immersed into the liquid sample, testing can still be completed in other testing areas that are not immersed in the liquid.

In some embodiments, the test strip 700 has two areas, that is, one area is a first area 706 with the testing area, and the other area is a second area 707 without the testing area, which are respectively provided on two ends of the testing area. The test strip 700 includes a first end 709 and a second end 708, the first area is close to the first end of the test strip, and the second area is close to the second end of the test strip. Substantially, when the test strip is inserted into the detection chamber, the first area is close to the opening 201 of the detection chamber, and the second area is located at the bottom of the detection chamber; therefore, the second area 707 is allowed to be in contact with the liquid even if there is the liquid at the bottom of the detection chamber. Since the test strip is supported by the non-absorbent material and the second area is the non-absorbent area, the testing area will not be in contact with the liquid when the test strip is inserted.

### Carrier of test strip

In addition that the test strip itself is used to contact with the liquid in the detection chamber 200 to test the presence or absence of the analyte in the liquid sample, and in some preferred embodiments, the testing element can also be provided on the carrier 300, one groove 305 is provided in the carrier, and the testing element is located in the groove. The carrier of the invention does not have the test strip at the beginning, and the test strip is inserted into the groove of the carrier 300 for testing or assaying when testing is performed. The carrier is different from those ordinary carriers that have test strips at the beginning and are inserted into the liquid when testing needs to be performed. Therefore, the carrier 300 of the invention has a direction to guide the insertion of the test strip, and also has the function to fix the test strip in a stable position after the test strip is inserted.

The test strip of the invention is sealed in a bottle at the beginning, and a plurality of test strips are provided in the invention, for example, 100-200 test strips; one of the test strips is taken out of the bottle and inserted into the detection chamber 200 for testing when testing needs to be performed. For example, in some embodiments, the liquid sample can be collected in the sample chamber 100, and the detection chamber 200 is in fluid communication with the sample chamber. When the liquid sample is collected by the sample chamber, a part of the liquid is also accommodated in the detection chamber 200, and then one test strip such as the test strip 700 is inserted into the detection chamber for testing. During detection, the sample chamber is sealed with the second cover 400 and the opening 201 of the detection chamber is sealed with the first cover 600. In this case, the test strip is located in the detection chamber 200, and the detection chamber is inverted or inclined to allow the liquid to contact with one or more testing areas on the test strip 700; after such contact is completed, the liquid sample is allowed to depart from the testing area, and the results of the testing areas are read with naked eyes; for example, the color is observed for shade and saturation and compared with the standard colourimetric card to judge the testing results to be positive or negative.

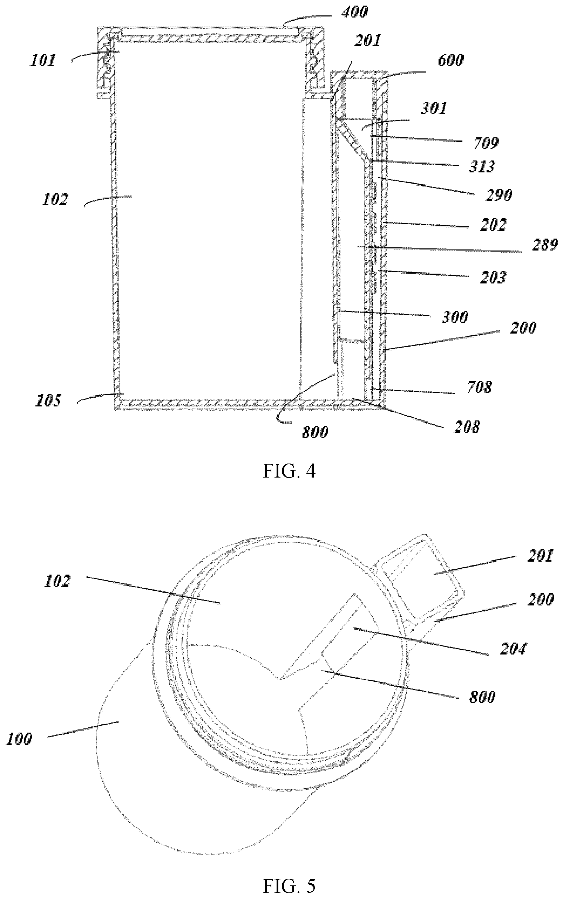

In a preferred embodiment of the invention, the detection chamber 200 is provided with the carrier 300 in advance, the carrier has a groove 305, and the test strip is allowed to lean against or be located in the groove 305. The groove has an opening which faces the transparent side wall of the detection chamber; and when the test strip is inserted, the testing area also faces the transparent side wall, such that the testing results are conveniently observed during testing. In some embodiments, the carrier of the invention includes a groove 305, where the groove is surrounded by a flat bottom and walls 306,307 on both sides to form a long and narrow channel. However, the test strip is located in the groove. Although the test strip is not adhered to the groove, the test strip can be avoided from falling off the groove during the later test. In the test process, the detection chamber 200 needs to be inclined, inverted, or shaken, such that the liquid is allowed to contact with different testing areas on the test strip and then is allowed to depart from the testing areas after contacting with such testing areas. In this case, the liquid moves violently during the test, and the test strip itself is very light. During the flow of the liquid, it is desired that the position of the test strip will not change, and preferably, the test strip is allowed to be stably located in the groove without falling off the carrier. In some embodiments, two support frames 304, 308 are provided on the back of the groove and lean against an inner wall 204 of the detection chamber, while the groove faces an outer wall 202 of the detection chamber, and the space 203 in the detection chamber is used for receiving the liquid sample from the sample chamber. The support frames 304,308 are used to make the carrier stably located in the detection chamber 200 in time. In some embodiments, the carrier is also provided with a diversion chamber 301 that is an inlet for inserting the test strip. In some embodiments, the diversion chamber 301 has a slope 302; the slope forms a funnel-shaped structure with other walls 303, 310, 302 of the diversion chamber, and the bottom of the funnel-shaped structure has an opening, and the opening 313 corresponds to the inlet of the groove 305, such that the opening can guide the test strip directly into the groove (for example, as shown in FIG. 4) when the test strip is inserted. A mark, such as an arrow, can be made on the test strip to indicate a direction in which the test strip is inserted into the carrier. This prevents the test strip from being inserted in a wrong direction. Generally, the testing area is not distributed at the center of the test strip, but close to one end thereof; a longer blank area is reserved at the other end thereof, such that the blank area is downward and close to the bottom of the detection chamber, and a shorter end thereof is located near the opening 201 of the detection chamber 200. In some embodiments, when the test strip is inserted into the groove, a part of the test strip is located in the diversion chamber 301 (one end 709 of the test strip as shown in FIG. 1), such that the diversion chamber also has the function to fix the test strip. After all, the width and thickness of the inlet 313 are substantially equivalent to those of the test strip, so as to prevent the test strip from falling off the groove in a subsequent test operation. Herein, by virtue of the surface 303 of the diversion chamber and the inlet 313, the test strip is located in the groove 305, and is in a relatively fixed position and is not easy to fall off from the groove or the carrier.

### Analyte

Examples that can use an analyte related to the invention include some small-molecule substances, including drugs (such as drug of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzodiazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The testing device of the invention may also be used for detecting drugs belonging to a medical use but easy to be taken excessively, such as tricyclic antidepressants (imipramine or analogues) and acetaminophen. These drugs are metabolized into micromolecular substances after absorbed by human body. These micromolecular substances exist in blood, urine, saliva, sweat and other body fluids or in some body fluids.

For example, the analyte detected by the invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Chemical analysis of any other clinical urine may be performed by lateral flow detection in combination with the device of the invention. An appropriate testing element according to the invention can be used to detect any analyte. Preferably, the testing device of the invention is used to detect small drug molecules in saliva and urine. Preferably, the testing device can be used to detect small molecular substances such as viruses and bacteria in saliva, throat or nasal fluid. The testing device can also be used to detect white blood cells, red blood cells, urobilinogen, urine vitamin c, urine crystals, urine specific gravity, urine albumin, urine acetone bodies, urine colony count, urine pH and nitrous acid in the liquid samples. For example, the testing device can also be used to detect the levels of white blood cells, red blood cells, urobilinogen, urine vitamin c, urine crystals, urine specific gravity, urine albumin, urine acetone bodies, urine colony count, urine pH and nitrous acid.

The analyte in the urine can be tested by an immune method or a chemical method. Testing by the chemical method means that the absorbent material is treated with chemical substances. When the urine contains a specified amount of a specific analyte, a chemical reaction will occur on the absorbent material, and colored substances are produced and make the absorbent material colored. Through comparison of the colored substances and a standard colorimetric card, it is possible to know whether the analyte in the urine exists or how much it probably exists. Generally, the thick color indicates the high content of the analyte. For example, when the nitrous acid is tested, aromatic sulfadiazine is treated on the testing area and reacts with the nitrous acid to generate a diazo compound; the diazo compound reacts with 2,3,4-tetrahydrobenzo (h) quinoline -3-phenol to generate a pink color substance; and the pink color substance precipitates on the testing area, such that the testing area shows pink. The white blood cells in urine contain esterase that can catalyze the hydrolysis of privatized pyrrole amino acid ester to release 3-hydroxy-5-phenylpyrrole. Then, the pyrrole reacts with diazonium salt to form purple. The reaction is used to test the content or number of the white blood cells in urine.

### Testing device

The testing device refers to a device for detecting the presence or absence of an analyte in a sample. The collection device refers to a device for collecting and storing liquid samples. The testing device can include a collection device, and the collection device can also include the testing device, or the collection device is separated from the testing device; during the test, the collection device and the detection device are combined to complete the test. Alternatively, the collection device and the detection device are of an integral structure, and therefore the collected liquid sample can be tested immediately to obtain the testing result, and at the same time, the test sample can be separated from the collected sample, such that secondary test can be performed (if necessary). The testing device and the detection chamber here are interchangeable, and the collection device and the collection cavity are also interchangeable, but only their functions are interchanged depending on their different roles. For example, the collection device of the invention may not include the detection chamber, but the testing element or the carrier with the testing element can be inserted into the collection device; the collection device including the testing element may also be called the testing device, for example, the collection chamber 100 includes the testing element. Of course, the collection device can include a space where the testing element is provided, but does not necessarily include the testing element, and the testing element can be combined with the collection device at any suitable time to serve as the testing device. For example, the collection device may include a space for accommodating the testing element, such as the detection chamber; alternatively, the liquid collection chamber of the collection device has a suitable position where the testing element or the carrier including the testing element is provided.

### Sample chamber and detection chamber

The testing device herein includes the sample chamber 100 and the detection chamber 200. The first cover is used for sealing the opening 201 of the detection chamber, and the second cover 400 is used for sealing the opening 101 of the sample chamber. The carrier 300 is in advance provided in the detection chamber. In this case, the whole device includes no testing elements, the detection chamber includes no testing elements, and the detection chamber is in fluid communication with the sample chamber. In some embodiments, the collection chamber 100 herein is a chamber for receiving the liquid sample and used for receiving samples, such as urine samples, as shown in FIG. 1 and FIG. 4. The collection herein can be that a test subject directly allows the liquid sample to flow into the sample chamber 100, or an operator adds the liquid sample into the sample chamber 100 through other tools, such as a pipette or other containers. Therefore, the sample chamber 100 has an internal space 102, a bottom 105, and a chamber enclosed by side walls 106 connected with the bottom, and can be transparent or opaque. In some embodiments, the sample chamber has an opening 101 for receiving the liquid sample, and the detection chamber also has an opening 201 for inserting the test strip. The detection chamber also has a bottom 208, a channel 800 is provided near the bottom, and the sample chamber is in fluid communication with the detection chamber through the channel. The liquid sample is allowed to flow into the sample chamber 102 during the collection, and a part of the liquid flows into the detection chamber 200 through the channel 800. In this case, if the detection chamber does not have the first cover 600 to seal the opening 201 of the detection chamber, the sample chamber shares a liquid level with the detection chamber. Although the detection chamber and the sample chamber have a different liquid level, they have a same liquid level. In this case, the test strip 700 is inserted into the diversion chamber 301 of the carrier 300 through the opening 201 of the detection chamber and then inserted into the groove 305 through the inlet 313, and the first cover 600 seals the opening 201 of the detection chamber and the second cover 400 seals the opening 101 of the sample chamber. In this case, the test strip is located in the detection chamber 200, and the liquid level of the detection chamber is below the testing area, for example, the second area is close to the bottom of the detection chamber, and the testing area remains dry. The testing device is inverted, the second cover is allowed to stand on a plane, and the bottom 105 faces upward. In this case, the liquid in the detection chamber flows from the bottom 208 to the opening 201, forming a liquid level at the opening. In this case, the testing area on the test strip is immersed in the liquid; after 1-10 seconds, the device is inverted again and returned to the initial state, and the liquid in the detection chamber is returned to the bottom 208 when testing is performed, such that the testing area can absorb the liquid sample and makes reaction, the color in the testing area is observed for change or shade and compared with the standard colourimetric card to obtain the testing results.

In some embodiments, the first cover and the second cover seal the detection chamber and the sample chamber in sequence. For example, in the above embodiments, the opening 201 of the detection chamber is not sealed by the first cover 600 when the liquid sample is collected by the sample chamber 100. If the opening 201 of the detection chamber is sealed by the first cover, the liquid sample is collected by the sample chamber 100. Since the opening 201 of the detection chamber is sealed, the liquid level at the bottom of the detection chamber is just above the height of the channel. This is due to the fact that a section of air is sealed by the liquid in the detection chamber, and the sum of the pressure in the air and the pressure of the liquid level is balanced with the pressure in the sample chamber, so the liquid cannot flow into the detection chamber again. Therefore, the height of the channel between the detection chamber and the sample chamber directly determines the liquid level of the detection chamber. Therefore, in such a way, the height h of the channel 800 can be provided at a position below the lowest testing area 705 on the test strip, the liquid level of the detection chamber is substantially equal to the height of the channel, the device (the second cover seals the collection chamber) is still inverted or inclined when testing is performed, the liquid in the detection chamber flows from the bottom to the vicinity of the opening 201, a liquid level is gathered at the opening as the bottom, and the liquid contacts with the testing area of the testing element and then is allowed to depart from the testing area (the testing device is inverted again). Essentially, a section of the liquid in the channel essentially flows from the bottom to the opening when the device is inverted, such that a section of the liquid with a height is formed at the opening. No matter whether one or more testing areas are immersed in the liquid sample, the volume of the liquid sample remains unchanged, which can achieve the purpose of volume quantification.

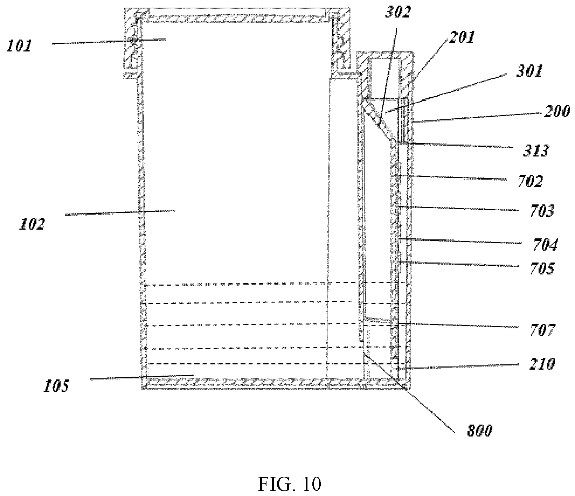

In some embodiments, when the liquid sample is collected in the sample chamber 100 and the opening 101 of the sample chamber is sealed by the cover, generally, the liquid in the sample chamber has an appropriate level, for example, it cannot be higher than a maximum level 103. Of course, a minimum level can also be specified. The purpose of the maximum level is to hope that the level of the liquid in the detection chamber cannot be higher than the testing area (after the test strip is inserted into the detection chamber), and the liquid contacts with the testing area in advance so as to make reaction in advance. Moreover, when the detection chamber is inverted again after inverted, the testing area is still immersed or in contact with the liquid, resulting in false negative results. The lowest level is to ensure that the liquid sample has the lowest level in the detection chamber 200. When the detection chamber is inverted, all the testing areas can be immersed in the liquid sample in the detection chamber, such that each testing area can contact or react with the liquid. Actually, the liquid level of the detection chamber should not be higher than the lowest testing area at the beginning, for example, as shown in FIG. 10, it should not be higher than the testing area 705, but when testing is performed, it is desired that the liquid can contact with all the testing areas 702, 703, 704, 705. A simple way is that the device is inverted, and the liquid level of the detection chamber can be higher than the testing area 705. As mentioned above, the testing areas on the test strip are located near one end of the test strip, instead of being located at the center of the test strip. Generally, the testing area 706 is allowed to be close to one end of the detection chamber 200, and a more blank area 707 is reserved at the other end of the test strip.

In some embodiments, because the space of the detection chamber is relatively small and the carrier 300 is located in the space, and when the test strip is inserted into the groove 305 of the carrier 300, actually, a distance between the test strip on the carrier and the outer wall 202 of the detection chamber is very short (almost 2-3 mm). Actually, in the detection chamber, the groove 304 of the carrier and the wall of the detection chamber 200 form a long and narrow gap 290. A narrow space 289 is also formed between the back of the groove 305 and the side wall 204 of the detection chamber 200. When the testing device is inverted, it is desired that the liquid sample will flow into the narrow slit 209 to contact with the testing area on the test strip. Therefore, there is a specified distance between the tail end of the groove 305 in the carrier 300 and the bottom of the detection chamber 200, and a hole 210 is reserved such that the liquid can be allowed to flow from the hole 210 to the narrow slit 290 to contact with the testing area on the test strip 700. After contact, it is desired that the liquid will depart from the testing area (for example, the device is inverted again) so as not to affect the final testing results due to the presence of the liquid near the testing area (under the surface tension of the liquid). Therefore, after the device is inverted, it is found that there is still the liquid sample in the narrow slit 290. In this case, the sample chamber can be inclined, such that all the liquid at the bottom of the detection chamber can flow into the sample chamber through the channel 800. Therefore, the residual liquid will flow to the bottom 208 of the detection chamber 200 and can also flow into the sample chamber 100. Technically, the liquid sample is collected in the sample chamber, but some air is still left in the sample chamber, for example, the air is sealed between the maximum liquid level and the second cover. When the first cover 600 is removed from the opening 201 of the detection chamber, the liquid level of the detection chamber is substantially the same as that of the sample chamber. When the test strip 700 is inserted into the detection chamber through the opening 201 and then the opening of the detection chamber is sealed with the first cover, the air is actually sealed at one end of the detection chamber. When the liquid sample flows between the sample chamber and the detection chamber, sometimes the volume of the liquid sample in the sample chamber increases and the volume of the liquid sample in the detection chamber decreases; or the volume of the air in the sample chamber changes with the increase in the volume of the liquid in the detection chamber and the decrease in the volume of the liquid in the sample chamber. The total volume of the liquid is constant, only the inclination and inversion speed of the device will affect the distribution of the liquid sample between the two chambers. For example, when the device is inverted, it is desired that the more liquid sample will flow into the detection chamber to contact with the testing area. The inclination angle of the device is changed, such that more air flows from the detection chamber 200 to the sample chamber 100, and more liquid will flow into the detection chamber. After the liquid sample is out of contact with the testing area on the test strip in the detection chamber, it is desired that the liquid will depart from the testing area, and the liquid will be accommodated in the detection chamber as much as possible and at least not in the area of the narrow slit 290 of the detection chamber, to avoid continuing to contact with the testing area. In this case, the liquid is allowed to flow into the sample chamber as much as possible, and the channel 800 of the sample chamber can be completely exposed, such that more air can flow into the detection chamber to realize the exchange of gas between the detection chamber and the sample chamber. In this case, the liquid will smoothly flow into the sample chamber 100, and the liquid staying in the narrow slit will flow to the bottom 208 of the detection chamber or flow into the sample chamber.

In other embodiments, if an amount of the liquid in the sample chamber is very small during collection and if all the liquid flows into the detection chamber, a liquid level is formed only in the detection chamber and also below the testing area 705. In this case, the volume of the sample chamber is greater than or far greater than that of the detection chamber; although the two chambers have a same liquid level, the diameters of the cross sections are very different; therefore, when the volume of the liquid collected by the sample chamber is very small, all the liquid can flow into the detection chamber through the channel 800; for example, the sample chamber is inclined towards the direction of the detection chamber when inverted, such that the liquid can flow into the detection chamber; then, the detection chamber is slowly inverted, such that all the liquid can flow into the detection chamber 200 and the testing area is immersed in the liquid sample. Therefore, the minimum amount of the liquid collected is the volume from the opening of the detection chamber to the height of the testing area 705 of the testing element when all the liquid is allowed to flow into the detection chamber.

In some embodiments, there is a surrounding label (omitted) on the outer wall of the sample chamber 100 near the bottom. The main function of the label is to record the information of the test subject, such as the time of collecting the sample, the name or number of the test subject. In some embodiments, the label is also provided with marking lines for the minimum liquid level and the maximum liquid level. This indicates the minimum liquid level and the maximum liquid level for the sample chamber 100, which is actually the amount of the liquid. This is a label indicating the maximum amount of the liquid sample and the minimum amount of the liquid sample. However, in some cases, if the test subject directly urinates into the sample chamber 100 during the test, the urine often exceeds the maximum labeling level. In this case, setting of the testing area on the test strip (for example, the test strip is used as shown in FIG. 7) can avoid some potential problems, which will be explained again with specific examples. In some embodiments, the outer walls of the bottom of the whole chamber are not surrounded by the label, but are reserved with a window not covered by the label; the level or state of the liquid sample can be observed through the transparent window, and this will be described in detail later. Generally, the label is opaque and the sample chamber can be transparent, so the liquid in the sample chamber is conveniently observed through the window. In some embodiments, the detection chamber 200 is located in the outer wall of the sample chamber, and the detection chamber and the sample chamber share a wall, and a channel 800 is provided at the bottom of the wall near the two chambers, such that the two chambers can form fluid communication.

### Embodiments

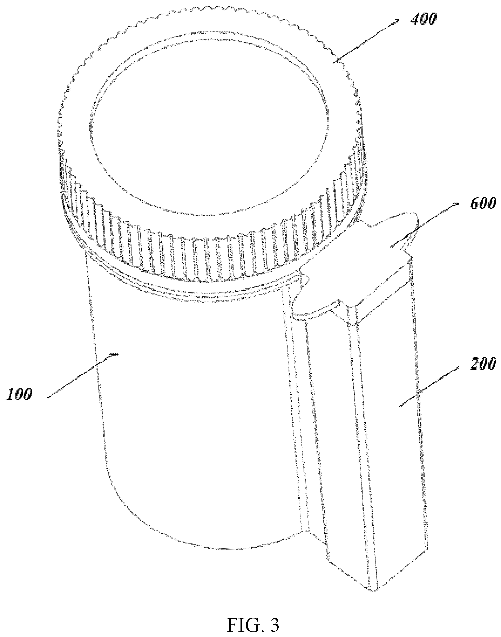

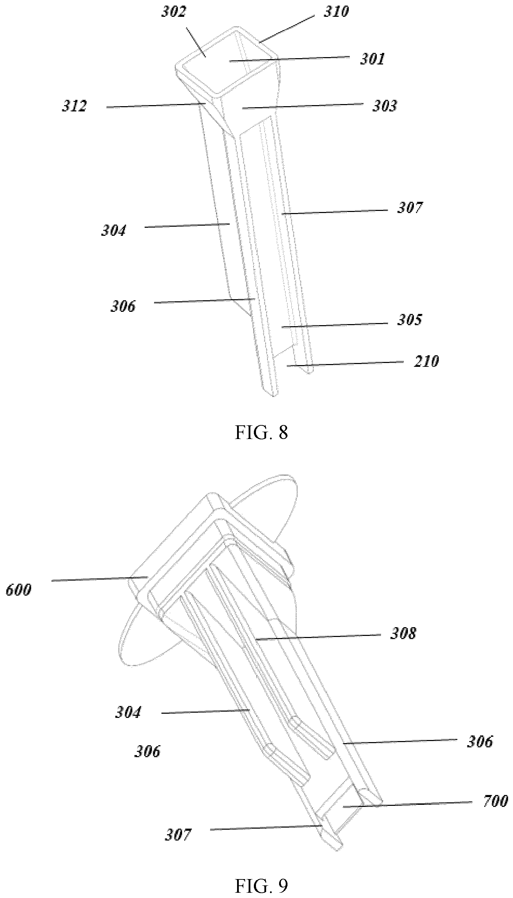

As shown in FIG. 1 - FIG. 13, the invention provides a testing device, and the device includes a sample chamber 100, where the sample chamber 100 has an opening 101, a cylindrical bottom 105, and a cover 400 to seal the opening; one detection chamber 200 is provided outside the sample chamber and has a bottom 208, an opening 201, and a cover 600 to seal the opening. A carrier 300 is provided inside the detection chamber and has a groove 305 that is a wall 202 facing the front side of the detection chamber, and the carrier is provided with a diversion chamber 301 near the opening 201 of the detection chamber 200; the bottom of the diversion chamber 301 has an inlet 313, and the diversion chamber and the inlet 313 are of an area where the test strip is allowed to be inserted; moreover, the inlet is the same as the groove 305, the test strip is directly located in the groove when inserted into the detection chamber, and the test strip is usually longer than the groove. In addition, the tail end of the carrier is close to the bottom of the detection chamber, but does not contact with the bottom. There is a height between the tail end and the bottom of the detection chamber, such that a hole 210 is formed; and there is a channel 800 at the bottom of the detection chamber and the bottom of the sample chamber.

A test strip 700 is provided, and four testing areas 702, 703, 704, 705 are provided at one end 709 of the test strip and arranged within the area in sequence from the end 709, while a blank area is reserved at the other end 708 of the test strip without the testing area; in addition, the testing areas are all arranged on an absorbent sheet made of plastic cloth. A plurality of test strips are sealed in a bottle, and the detection chamber and the sample chamber do not include the test strips.

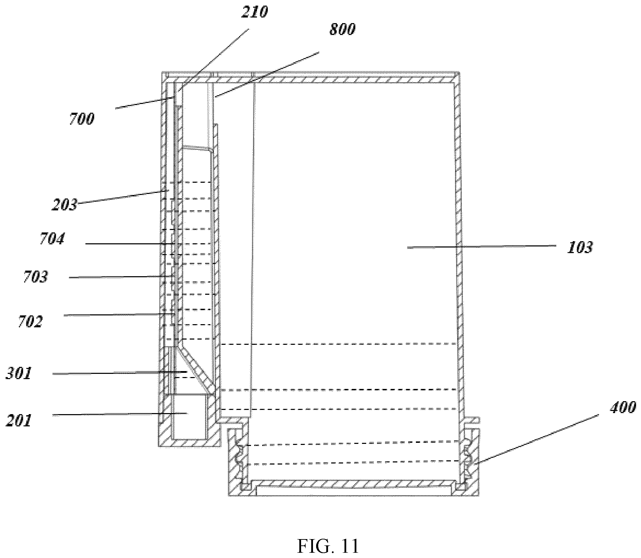

When testing needs to be performed, the cover 400 of the sample chamber is removed, and the liquid chamber is allowed to collect the liquid sample; for example, the urine sample is allowed to be located in the sample chamber; in this case, the cover 600 of the detection chamber can be removed, such that the liquid in the sample chamber also can flow into the detection chamber; the two chambers have the same liquid level, and the liquid is located between the maximum liquid level and the minimum liquid level. In this case, the cover 400 of the sample chamber can be covered, a test strip can be taken out of the bottle, and one end 708 of the test strip is allowed to enter the diversion area 301, is inserted into the groove 305 of the detection chamber through the opening 313 in the bottom, and stands in the detection chamber 200. In this case, as shown in FIG. 10, the blank area of the second end of the test strip is located in the liquid, but the lowest testing area 705 is located above the liquid level, the cover of the detection chamber 200 is covered, and the opening 201 of the detection chamber is sealed. The detection chamber is inverted (the sample chamber is inverted), such that the liquid in the detection chamber flows to the opening 201 of the detection chamber and a liquid level is formed at the opening. In this case, the four testing areas on the test strip 700 are all in the liquid and absorb the liquid. After inverted for 10 seconds (as shown in FIG. 11), the detection chamber is inverted again to return to a state as shown in FIG. 10, and the liquid in the detection chamber flows to the bottom and forms a liquid level at the bottom. In this case, the change in the color of the testing area needs to wait within 3 minutes, a standard colourimetric card is provided and has a color block, on which the testing results are marked as being positive or negative according to the shade of color. If it is found that the color of the testing area does not change, the testing results are considered to be negative; and when it is found that the color of the testing area is the same as the corresponding positive test color block on the test strip, the testing results are positive.

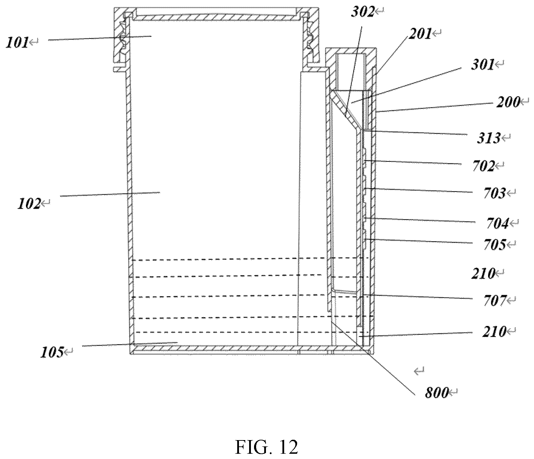

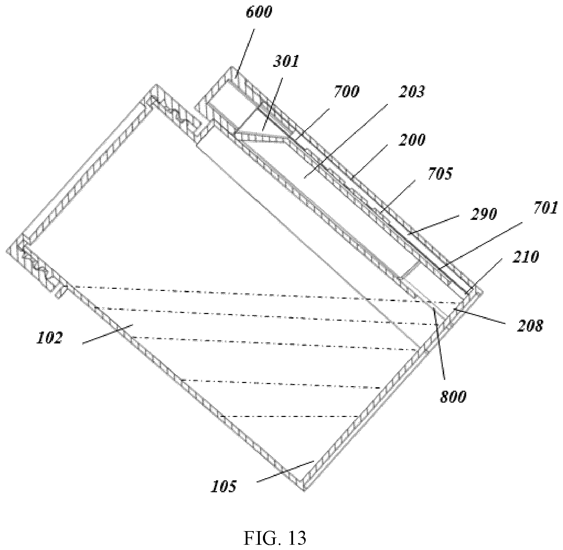

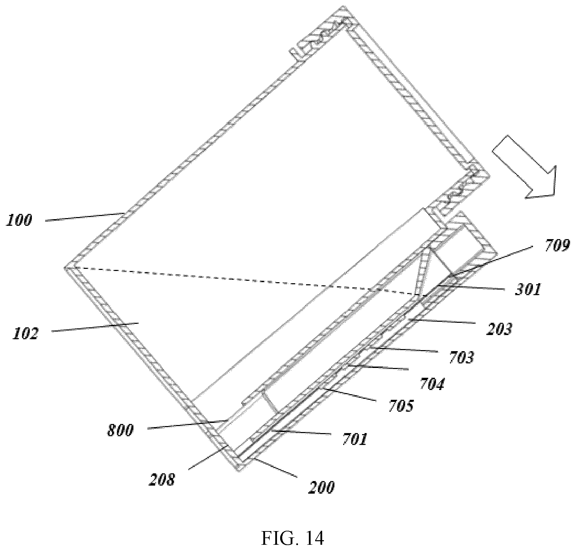

In some embodiments, the detection chamber may be inclined or the sample chamber may be inclined, such that the liquid in the detection chamber contacts with the testing area. A preferred inclination direction is that the detection chamber is inclined in a direction where the detection chamber is located. For example, as shown in FIG. 14, if the detection chamber is located at a side of the sample chamber and is inclined towards the position where the detection chamber 200 is located, the liquid can be used to fill the detection chamber or at least immerse the testing area on the test strip, the detection chamber is allowed to return to an upright position after inclined for a period of time, as shown in FIG. 12. In this case, the liquid returns to the bottom of the detection chamber, but departs from the testing area. The testing area is no longer in contact with the liquid, such that the analyte (if the analyte contains or exceeds a concentration level) in the sample on the testing area reacts with the substance on the testing area to produce color. The color is compared with the color block on the provided standard colourimetric card to judge the testing results to be negative or positive. The so-called "direction towards the detection chamber" means that the detection chamber is fully filled with the liquid or the liquid at the bottom of the detection chamber can flow back to the opening of the detection chamber, such that the liquid contacts with the testing area on the test strip when the liquid is filled into the detection chamber or flows back. As shown in FIG. 10 and FIG. 14, the inclination direction is to allow the detection chamber to be inclined, the position of the test strip in the detection chamber is relatively fixed and the test strip is inclined with the detection chamber; in an inclination process, the detection chamber is gradually filled with the liquid to contact with the liquid sample. For another example, as shown in FIG. 13, if the detection chamber is inclined towards its opposite direction (FIG. 13) from an upright direction as shown in FIG. 10, the liquid in the detection chamber will flow to the sample chamber, and the testing element is still dry and cannot be in contact with the liquid sample, so testing cannot be completed. Herein, FIG. 13 is used to explain the inclination direction. In some embodiments, after the test sample contacts with the testing area, the liquid in the detection chamber is allowed to depart from the detection chamber as far as possible by a way in FIG. 13, instead of staying in the testing area, and especially the liquid staying in the narrow slit 290 is allowed to flow out of it.

The following specific embodiments are also a part of the invention.

A device for testing an analyte in a liquid sample is provided, and the device includes: a sample chamber for receiving the liquid sample, where the sample chamber includes an opening and side walls enclosing a chamber; a detection chamber, where the detection chamber is in fluid communication with the sample chamber, the detection chamber is provided with the opening through which a testing element is inserted into the detection chamber, and the testing element has a testing area; and a first cover, where the first cover is used for sealing the opening of the detection chamber.

In some embodiments, the testing element includes one or more testing areas with a water absorption capacity, and the testing areas are spaced apart on a non-absorbent carrier. In some embodiments, the liquid cannot flow between the testing areas or the liquid cannot flow between the testing areas due to the existence of the non-absorbent carrier. In some embodiments, the testing element has a first state and a second state in the detection chamber; when the testing element is inserted into the detection chamber, the testing area is not in contact with the liquid sample or the liquid does not contact with the testing area; and when the testing element is in the second state, the testing area is in contact with the liquid sample or the liquid sample directly contacts with the testing area. In some embodiments, the testing area further includes a third state; after an operation in the second state is completed, the testing area is in the third state, and the testing area departs from the liquid sample or the liquid sample departs from the testing area in the third state. In some embodiments, the testing area is dry in the first state; and when the testing area is in the second state and the third state, there is a part of the liquid sample on the testing area. In some embodiments, the detection chamber includes the testing element before the initial test, and the test strip is inserted into the detection chamber only when testing needs to be performed. In some embodiments, when the test strip is inserted into the detection chamber, testing is not immediately performed in the testing area, and in this case, there is no the liquid sample on the testing area. In some embodiments, when the testing device starts to collect the liquid with the sample chamber, the detection chamber does not include the testing element therein. In some embodiments, the channel between the detection chamber and the sample chamber has a height; when the testing element is inserted into the detection chamber, the height is lower than the testing area that is located on the testing element and closest to the bottom of the detection chamber.

In some embodiment, a carrier against which the testing element is allowed to lean is provided in the detection chamber; and when the testing element is inserted into the detection chamber, the testing element leans against the carrier. In some embodiments, a groove is provided in the carrier, and the testing element is located in the groove. In some embodiments, the carrier includes one diversion chamber, through which the test strip can be inserted into the groove. In some embodiments, the bottom of the diversion chamber is provided with an opening, and the test strip is diverted into the groove through the opening. In some embodiments, the groove faces a transparent front wall of the detection chamber. In some embodiments, when the test strip is inserted into the groove, the testing area faces the transparent front wall of the detection chamber. In some embodiments, when the test strip is inserted into the carrier, a part of one end of the test strip is located in the diversion chamber, thereby keeping the test strip stably located in the groove without being detached.

In some embodiments, the detection chamber is located outside the sample chamber, and there is an opening at a bottom of the detection chamber and a bottom of the sample chamber; and when the sample chamber is used for collecting the liquid sample, the liquid sample in the sample chamber flows into the detection chamber through the opening at the bottom. In some embodiments, when the liquid sample is accommodated in the sample chamber, a part of the liquid sample is located in the detection chamber; and when the testing element is inserted into the detection chamber, the testing area of the testing element is not in contact with the liquid sample. In some embodiments, when the liquid sample is accommodated in the sample chamber, a part of the liquid sample is accommodated in the detection chamber; and when the testing element is inserted into the detection chamber and the testing device is inverted, the liquid sample in the detection chamber contacts with the testing element to allow the testing area to be in the second state. In some embodiments, there are substances capable of directly or indirectly reacting with the analyte in the liquid sample on the testing area, such that colored substances are generated and precipitated on the testing area. In some embodiments, the device further includes a second cover for sealing the opening of the sample chamber.

The invention provides a method for testing an analyte in a liquid sample, and the method includes: providing a device, where the device includes a sample chamber for receiving the liquid sample, and the sample chamber includes an opening and side walls enclosing a chamber; a detection chamber, where the detection chamber is in fluid communication with the sample chamber, and the detection chamber is provided with the opening through which a testing element is inserted into the detection chamber; allowing the sample chamber to collect the liquid sample such that a liquid flows into the detection chamber; and inserting a testing element into the detection chamber, where during the insertion of the testing element, the liquid does not flow along the testing element, or there is no the liquid sample on the testing area, or the testing area is unable to contact with the liquid sample. In some embodiments, the opening of the detection chamber is sealed with a first cover, or the opening of the sample chamber is sealed with a second cover; or the opening of the detection chamber is sealed with a first cover and the opening of the sample chamber is sealed with a second cover; or the openings of the detection chamber and sample chamber are sealed by one cover. Then, the liquid in the detection chamber is allowed to contact with the testing area on the test strip for a suitable period of time, for example, 1-30 seconds, 1-60 seconds, 5 seconds and 10 seconds, such that the testing area can absorb a sufficient amount of the liquid or a part of the liquid. Then, the liquid is allowed to immediately depart from the testing area or the liquid is allowed to be immediately separated from the testing area, thereby allowing the testing area to absorb the liquid sample. In some embodiments, there are many ways to allow the liquid to contact with the testing area or depart from the testing area after the liquid contacts with the testing area. Such ways may be that after the liquid sample is collected, the detection chamber is inverted and inclined, or inverted and then inclined, or inclined and then inverted. Substantially, the liquid is allowed to flow in the detection chamber, and in a flowing process, the liquid can contact with or depart from the testing area. In some preferred embodiments, the detection chamber is inverted, such that the liquid at the bottom of the detection chamber flows to the opening of the detection chamber, and the testing area is immersed in the liquid sample for a period of time.

In some embodiments, when the testing element is inserted into the detection chamber through the opening of the detection chamber, the opening of the detection chamber is sealed by the first cover; and then the liquid sample is allowed to contact with the testing area for a specified time, the liquid sample is allowed to depart from the testing area. In some embodiments, after the liquid sample contacts with the testing area, the testing area is allowed to be moistened. In some embodiments, the testing area is provided in one or more, and the testing area has a water absorption capacity and is provided on a non-absorbent carrier. In some embodiments, a method of allowing the liquid sample to contact with the testing area includes inverting the testing device. In some embodiments, after the liquid sample is allowed to contact with the testing area, the liquid sample is allowed to depart from the testing area by returning the testing device to an upright state again.

All the patents and publications mentioned in the description of the invention indicate that these are public technologies in the art and can be used by the invention. All the patents and publications cited herein are listed in the references, just as each publication is specifically referenced separately. The invention described herein can be realized in the absence of any one element or multiple elements, one restriction or multiple restrictions, where such restriction is not specifically described here. For example, the terms "comprising", "essentially consisting of" and "consisting of' in each embodiment herein may be replaced by the rest 2 terms. The terms and expressions which have been employed herein are descriptive rather than restrictive, and there is no intention to suggest that these terms and expressions in this description exclude any equivalents, but it is to be understood that any appropriate changes or modifications can be made within the scope of the invention and appended claims.

## Claims

1. A device for testing an analyte in a liquid sample, comprising:
a sample chamber for receiving the liquid sample, wherein the sample chamber comprises an opening and side walls enclosing a chamber;
a detection chamber, wherein the detection chamber is in fluid communication with the sample chamber, the detection chamber is provided with the opening through which a testing element is inserted into the detection chamber, and the testing element has a testing area; and
a first cover, wherein the first cover is used for sealing the opening of the detection chamber.

2. The device according to claim 1, wherein the testing element comprises one or more testing areas with a water absorption capacity, and the testing areas are provided on a non-absorbent carrier.

3. The device according to claim 2, wherein a liquid is unable to flow between the testing areas or a liquid is unable to flow between the testing areas due to the existence of the non-absorbent carrier.

4. The device according to any one of claims 2-3, wherein the testing element has a first state and a second state in the detection chamber; when the testing element is inserted into the detection chamber, the testing area is not in contact with the liquid sample or the liquid does not contact with the testing area; and when the testing element is in the second state, the testing area is in contact with the liquid sample or the liquid sample directly contacts with the testing area.

5. The device according to claim 4, wherein the testing area further comprises a third state; after an operation in the second state is completed, the testing area is in the third state, and the testing area departs from the liquid sample or the liquid sample departs from the testing area in the third state.

6. The device according to claim 5, wherein the testing area is dry in the first state; and when the testing area is in the second state and the third state, there is a part of the liquid sample on the testing area.

7. The device according to any one of claims 1-6, wherein a carrier against which the testing element is allowed to lean is provided in the detection chamber; and when the testing element is inserted into the detection chamber, the testing element leans against the carrier.

8. The device according to claim 7, wherein a groove is provided in the carrier, and the testing element is located in the groove.

9. The device according to any one of claims 7-8, wherein the carrier includes an insertion hole for inserting the testing element into the groove.

10. The device according to any one of claims 1-9, wherein the detection chamber is located outside the sample chamber, and there is an opening at a bottom of the detection chamber and a bottom of the sample chamber; and when the sample chamber is used for collecting the liquid sample, the liquid sample in the sample chamber flows into the detection chamber through the opening at the bottom.

11. The device according to any one of claims 1-10, wherein when the liquid sample is accommodated in the sample chamber, a part of the liquid sample is accommodated in the detection chamber; and when the testing element is inserted into the detection chamber, the testing area of the testing element is not in contact with the liquid sample.

12. The device according to any one of claims 1-11, wherein when the liquid sample is accommodated in the sample chamber, a part of the liquid sample is accommodated in the detection chamber; and when the testing element is inserted into the detection chamber and the testing device is inverted, the liquid sample in the detection chamber contacts with the testing element to allow the testing area to be in the second state.

13. The device according to any one of claims 1-12, wherein there are substances capable of directly or indirectly reacting with the analyte in the liquid sample on the testing area, such that colored substances are generated and precipitated on the testing area.

14. The device according to any one of claims 1-13, wherein the device further comprises a second cover for sealing the opening of the sample chamber.

15. A method for testing an analyte in a liquid sample, comprising:
providing a device, wherein the device comprises a sample chamber for receiving the liquid sample, and the sample chamber comprises an opening and side walls enclosing a chamber;
a detection chamber, wherein the detection chamber is in fluid communication with the sample chamber, the detection chamber is provided with the opening through which a testing element is inserted into the detection chamber, and the testing element has a testing area; and
a first cover, wherein the first cover is used for sealing the opening of the detection chamber;
allowing the sample chamber to collect the liquid sample such that a part of the liquid sample flows into the detection chamber; and
inserting the testing element through the opening of the detection chamber, wherein after the testing element is inserted, the liquid sample is not allowed to contact with the testing area and the testing area is allowed to be kept dry.

16. The method according to claim 15, wherein when the testing element is inserted into the detection chamber through the opening of the detection chamber, the opening of the detection chamber is sealed by the first cover; and then the liquid sample is allowed to contact with the testing area for a specified time, the liquid sample is allowed to depart from the testing area.
